# EUROPÄISCHE PATENTSCHRIFT

(11) **EP 0 673 944 B1**
(45) Veröffentlichungstag und Bekanntmachung des Hinweises auf die Patenterteilung: **16.08.2001**
(21) Anmeldenummer: 95103403.2
(22) Anmeldetag: 09.03.1995
(51) Int. Cl.: C07F 9/53, C07C 17/14

(54) **Verfahren zur Herstellung von 2,2'-Bis(diphenyl-phosphinylmethyl)-1,1'-binaphthyl und neue Verbindungen aus dieser Stoffgruppe**
Process for preparing 2,2'-bis(diphenyl-phosphinylmethyl)-1,1'binaphtyl and novel compounds belonging to this group of substances
Procédé pour préparer des 2,2'-bis(diphényl-phosphinylméthyl)-1,1'-binaphtyles et nouveaux composés parmi ce groupe de substances

(30) Priorität: 23.03.1994 DE 4409973; 19.09.1994 DE 4433295
(43) Veröffentlichungstag der Anmeldung: 27.09.1995
(73) Patentinhaber: Celanese Chemicals Europe GmbH, 60439 Frankfurt am Main (DE)
(72) Erfinder: Kleiner, Hans-Jerg, Dr., D-61476 Kronberg (DE); Regnat, Dieter, Dr., D-65817 Eppstein (DE); Röschert, Horst, Dr., D-55437 Ober-Hilbersheim (DE)

(56) Entgegenhaltungen:
- EP-A- 0 595 150
- CHEMICAL ABSTRACTS, vol. 091, no. 11, 10. September 1979, Columbus, Ohio, US; abstract no. 091764, KUMADA M ET AL '2,2'-Bis(diphenylphosphino methyl)-1,1'-binaphthyl and its use as a ligand for asymmetric catalysts' & JP---7 939 059 (NITTO RIKEN INDUSTRIES, INC.;JAPAN) 24. März 1979
- TETRAHEDRON LETT. (TELEAY);77; (16); PP.1389-92, KYOTO UNIV.;DEP. SYNTH. CHEM.; KYOTO; JAPAN Tamao K et al 'Optically active 2,2'-bis(d iphenylphosphinomethyl)-1,1'-binaphthyl: a new chiral bidentate phosphine ligand for transition-metal complex catalyzed asymmetric reactions'

## Beschreibung

Die vorliegende Erfindung betrifft ein verbessertes Verfahren zur Herstellung von 2,2'-Bis(diphenylphosphinylmethyl)-1,1'-binaphthylen sowie neue Verbindungen aus dieser Stoffgruppe.
2,2'-Bis(diphenylphosphinylmethyl)-1,1'-binaphthyl (Formel I) stellt ein wichtiges Vorprodukt für die Herstellung von 2,2'-Bis(diphenylphosphinomethyl)-1,1'-binaphthyl (Formel II), einem zweizähnigen Phosphin (Phosphan) dar, das als Ligand für Katalysatoren, beispielsweise bei der durch Metallkomplexe katalysierten Kupplung von Halogenaromaten, Verwendung findet. 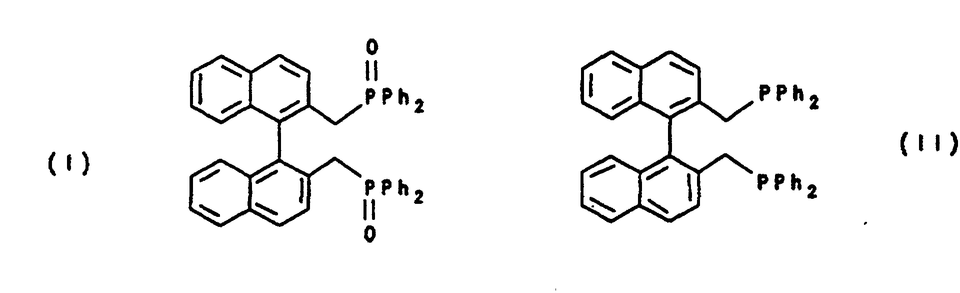 2,2'-Bis(diphenylphosphinylmethyl)-1,1'-binaphthyl läßt sich folgendermaßen herstellen. Man geht von der entsprechenden aromatischen Methylverbindung, nämlich von 2,2'-Dimethyl-1,1'-binaphthyl aus, bromiert die Methylgruppe mit N-Bromsuccinimid und erhält 2,2'-Bis(brommethyl)-1,1'-binaphthyl (Formel III). Bei dieser in der Literatur mehrfach beschriebenen, in Anwesenheit eines Radikalbildners, jedoch ohne Einwirkung von Licht durchgeführten Umsetzung findet in der Regel Tetrachlormethan als Lösungsmittel Anwendung (M. E. Jung et al., Tetrahedron Lett. 29 (1988) 6199; H. J. Bestmann et al., Chem. Ber. 107 (1974) 2926; J.-P. Mazaleyrat, Chem. Commun. 1985, 317; T. Hayashi et al., J. Am. Chem. Soc. 110 (1988) 8153).

Wegen des vergleichsweise niedrigen Siedepunkts von Tetrachlormethan, der bei 76,5 °C liegt, läßt sich eine Umsetzung bei höheren Temperaturen nur unter Druck durchführen, was einen erhöhten technischen Aufwand erfordert. Darüberhinaus liegt die nach diesem Verfahren erzielbare Ausbeute an 2,2'-Bis(brommethyl)-1,1'-binaphthyl mit ca. 60 % nicht sehr hoch.

Bei der Bromierung von 2,2'-Dimethyl-1,1'-binaphthyl entstehen neben dem gewünschten Wertprodukt (Formel III) im wesentlichen zwei Nebenprodukte, vermutlich 2-Brommethyl-2'-methyl-1,1'-binaphthyl und 2-Dibrommethyl-2'-brommethyl-1,1'-binaphthyl.

Die Isolierung von 2,2'-Bis(brommethyl)-1,1'-binaphthyl in reiner Form gelingt zwar durch Kristallisation oder durch Säulenchromatographie, muß aber einerseits durch erhebliche Ausbeuteverluste oder im Falle der Säulenchromatographie durch einen sehr hohen technischen Aufwand erkauft werden.

Die europäische Patentanmeldung EP 93 116 788.6, (EP-A-0595150), die unter anderem auf nicht veröffentlichte deutsche Patentanmeldung P 43 08 562.8 zurückgeht, beschreibt die Herstellung von 2,2'-Bis(brommethyl)-1,1'-binaphthyl durch Umsetzung von 2,2'-Dimethyl-1,1'-binaphthyl mit N-Bromsuccinimid in Gegenwart von Benzoylperoxid, jedoch ohne Einwirkung von Licht, in Chlorbenzol. Nach Beendigung der Umsetzung wird das Lösungsmittel verdampft, der Rückstand in Ethylacetat aufgenommen und zunächst mit 10 %iger Na₂SO₃-Lösung dann mit gesättigter Na₂CO₃-Lösung und abschließend mit gesättigter NaCl-Lösung gewaschen. Nach Trocknen und Umkristallisieren erhält man 65 % Ausbeute. Dieses Verfahren erweist sich jedoch als aufwendig (Abdampfen des Lösungsmittels, Überführung des Rückstandes in ein anderes Lösungsmittel und dreifache Wäsche jeweils mit einer wäßrigen Natrium-Salz-Lösung), zudem läßt auch die Ausbeute noch zu wünschen übrig.

Die Bromierung von 2,2'-Dimethyl-1,1'-binaphthyl führt generell zu einem Produktgemisch, dessen Handhabung problematisch ist. Es zeigt sich nämlich, daß sogar die unter schonenden Bedingungen (Vakuum) durchgeführte Abtrennung des Lösungsmittels zu einem Gemisch führt, das thermisch instabil ist und sich bereits ab einer Temperatur von lediglich 50 °C exotherm zu zersetzen beginnt.

Es ist aus Houben-Weyl, Methoden der organischen Chemie, Band V/4, Seiten 333 und 334 bekannt, daß Aralkylbromide in reinem Zustand farblose, thermisch meist wenig beständige Substanzen sind und manche von ihnen selbst im Vakuum nicht unzersetzt destillierbar sind. Naphthylmethylbromide sind im allgemeinen weniger beständig als Benzylbromide.

Die Isolierung des bei der Bromierung gebildeten Rohproduktes ist aus diesem Grund mit einem erheblichen Gefahrenpotential behaftet, das insbesondere bei den in technischem Maßstab in großen Mengen gehandhabten Einsatzstoffen bzw. Rohstoffen ein unberechenbares Risiko darstellt.

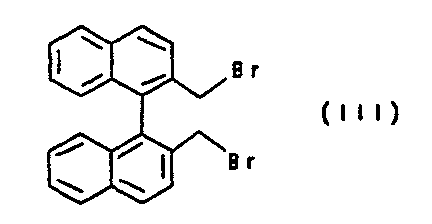

RO-P(Ph-(R')ₙ)₂ (IV)

Zur Herstellung von 2,2'-Bis(diphenylphosphinylmethyl)-1,1'-binaphthyl (Formel I) kann man mittels Arbusov-Reaktion reines 2,2'-Bis(brommethyl)-1,1'-binaphthyl - also kein Gemisch verschiedener bromierter Binaphthyle - mit Diphenylphosphinigsäure-methylester (Formel IV, wobei R für CH₃ und n für O steht) ohne Zusatz eines Lösungsmittels umsetzen. Wie aus JP 7 939 059 bzw. CA 91,91764 v hervorgeht, erhitzt man die beiden vorstehend genannten Komponenten im Verhältnis von 1:6,7 drei Stunden lang auf 130 °C und erhält das gewünschte Wertprodukt (Formel I). Die Umsetzung gestaltet sich allerdings problematisch, da, wie aus Experiment 4 der JP 7 939 059 zu entnehmen ist, es bei 87 °C zu einer explosionsartigen Reaktion kommt. Die erzielte Ausbeute beträgt lediglich 30 %. Das Verfahren eignet sich weder im Hinblick auf die erforderliche Prozeßsicherheit noch im Hinblick auf die Ausbeute für ein technisches Verfahren.

Es besteht daher ein Bedarf, ein Verfahren zur Herstellung von 2,2'-Bis(diphenylphosphinylmethyl)-1,1'-binaphthylen zu entwickeln, das die vorstehend genannten Nachteile nicht aufweist, sich darüberhinaus einfach und sicher ausführen läßt und das gewünschte Wertprodukt in hoher Ausbeute und Reinheit zugänglich macht.

Diese Aufgabe wird gelöst durch ein Verfahren zur Herstellung von 2,2'-Bis(diphenylphosphinylmethyl)-1,1'-binaphthylen. Es ist dadurch gekennzeichnet, daß man ein in einem Lösungsmittel durch Umsetzung von 2,2'-Dimethyl-1,1'-binaphthyl mit einem Bromierungsmittel hergestelltes, 2,2'-Bis(brommethyl)-1,1'-binaphthyl und weitere bromierte Binaphthyle enthaltendes Reaktionsgemisch, gegebenenfalls nach Abtrennung von nichtumgesetztem Bromierungsmittel, umgesetztem Bromierungsmittel und/oder Lösungsmittel, mit einem Diphenylphosphinigsäurealkylester der Formel (IV) RO-P(Ph-(R')ₙ)₂, worin R für einen Alkylrest mit 1 bis 5, insbesondere 1 bis 3 Kohlenstoffatomen, Ph für Phenyl, R' für einen Alkylrest mit 1 bis 4 Kohlenstoffatomen, CF₃, Fluor, Chlor oder Brom, insbesondere Methyl, CF₃ oder Fluor, und n für 0, 1 oder 2 steht bei 70 bis 180 °C, gegebenenfalls in Anwesenheit eines weiteren Lösungsmittels, umsetzt.

Ein Vorteil des erfindungsgemäßen Verfahrens besteht darin, daß man bei der Herstellung des Reaktionsgemisches auf eine Isolierung des 2,2'-Bis(brommethyl)-1,1'-binaphthyls (Formel III) verzichtet und auf diese Weise eine recht aufwendige Trennungsoperation, die zudem wegen der thermischen Instabilität des bei der Bromierung anfallenden Reaktionsproduktes ein Sicherheitsrisiko darstellt, einspart. Weiterhin kann - bei passender Wahl des Lösungsmittels - das in der Umsetzung verwendete Lösungsmittel in dem Reaktionsgemisch verbleiben. Es muß nicht aus dem Reaktionsgemisch entfernt werden.

Es ist jedoch auch möglich, das ursprünglich in der Stufe der Bromierung verwendete Lösungsmittel, falls erforderlich, durch ein weiteres Lösungsmittel zu ersetzen und das ursprünglich verwendete Lösungsmittel ganz oder teilweise abzudestillieren. Auf diese Weise tauscht man zwar das ursprünglich eingesetzte Lösungsmittel gegen das weitere Lösungsmittel aus, kann aber dennoch eine recht aufwendige Trennungsoperation (Isolierung des 2,2'-Bis(brommethyl)-1,1'-binaphthyl) vermeiden.

Das Reaktionsgemisch enthält - unabhängig von der Art der Bromierung - die üblicherweise bei einer Bromierung anfallenden bromierten Verbindungen, nämlich neben dem Wertprodukt im wesentlichen vermutlich die beiden zuvor erwähnten bromierten Nebenprodukte sowie das Lösungsmittel. In der Regel fällt ein Reaktionsgemisch an, das 60 bis 85 insbesondere 65 bis 83 Mol-% 2,2'-Bis(brommethyl)-1,1'-binaphthyl und 40 bis 15, insbesondere 35 bis 17 Mol-% weitere bromierte Binaphthyle enthält, wobei das noch vorhandene Lösungsmittel nicht berücksichtigt wurde.

Auf das eine Nebenprodukt, vermutlich 2-Brommethyl-2'-methyl-1,1'-binaphthyl, entfallen 1 bis 20, insbesondere 3 bis 18 Mol-% und auf das andere Nebenprodukt, vermutlich 2-Dibrommethyl-2'-brommethyl-1,1'-binaphthyl, 3 bis 20, insbesondere 5 bis 17 Mol-%. Die Art der Bromierung beeinflußt auch bis zu einem gewissen Grad sowohl die Menge der gebildeten bromierten Binaphthyle als auch das Verhältnis, in dem die beiden zuvor genannten Nebenprodukte gebildet werden.

Im allgemeinen spielt die verwendete Menge Lösungsmittel keine große Rolle. Sie sollte jedoch ausreichend bemessen sein. Im allgemeinen genügt es, 2,2'-Dimethyl-1,1'-binaphthyl und das Lösungsmittel im Gewichtsverhältnis 1:(3 bis 40), insbesondere 1:(4 bis 20), bevorzugt 1:(5 bis 15) zu verwenden.

Generell lassen sich Lösungsmittel, die unter den Reaktionsbedingungen der Bromierung inert sind, verwenden. Man kann ein einfach oder mehrfach chloriertes Benzol, einen einfach oder mehrfach chlorierten aliphatischen Kohlenwasserstoff, einen Ester einer aliphatischen Carbonsäure mit 1 bis 6 Kohlenstoffatomen und eines Alkylalkohols mit 1 bis 4 Kohlenstoffatomen oder Mischungen derselben als Lösungsmittel einsetzen. Beispiele hierfür sind Chloroform, Dichlormethan, Tetrachlormethan, Chlorbenzol, ortho-, meta- und para-Dichlorbenzol, Ameisensäuremethylester, Ameisensäureethylester, Essigsäuremethylester, Essigsäureethylester, Essigsäurepropylester, Propionsäuremethylester, Propionsäureethylester und Propionsäurepropylester. Als Lösungsmittel eignet sich gut Chlorbenzol oder Dichlorbenzol oder eine beliebige Mischung dieser Solventien. Besonders geeignet ist Chlorbenzol.

In einigen Fällen lassen sich auch Ester der vorstehend genannten Art, insbesondere Methylester und Ethylester aliphatischer Carbonsäuren mit 1 bis 3 Kohlenstoffatomen mit gutem Erfolg einsetzen.

Bei der Herstellung des Reaktionsgemisches kann man die Bromierung unter Einwirkung von Licht einer Wellenlänge von 10⁻⁵ bis 10⁻⁸ m, insbesondere 10⁻⁶ bis 2x10⁻⁷ durchführen. Hierbei ist es üblich, daß man die Umsetzung von 2,2'-Dimethyl-1,1'-binaphthyl bei -10 bis 75 °C ablaufen läßt. In einer Vielzahl von Fällen erweist es sich als ausreichend, 2,2'-Dimethyl-1,1'-binaphthyl mit dem Bromierungsmittel bei -5 bis 50, insbesondere 0 bis 40 °C umzusetzen.

Für die Bromierung kann man als Lichtquelle einen üblichen UV-Strahler, beispielsweise eine Tageslichtlampe, eine dotierte oder undotierte Quecksilberdampflampe oder Niederdruckquecksilberdampflampe verwenden. Andererseits kann man 2,2'-Dimethyl-1,1'-binaphthyl und das Bromierungsmittel in Abwesenheit von Licht, jedoch in Anwesenheit eines Radikalbildners bei 25 bis 150, insbesondere 40 bis 135 °C umsetzen.

Man kann die Bromierung mit einem üblichen Bromierungsmittel, beispielsweise Brom, N-Bromsuccinimid, 1 3-Dibrom-5,5-dimethylhydantoin, Brommeldrumsäure durchführen.

Es hat sich als günstig erwiesen, 2,2'-Dimethyl-1,1'-binaphthyl mit N-Bromsuccinimid als Bromierungsmittel umzusetzen. Man setzt üblicherweise 2,2'-Dimethyl-1,1'-binaphthyl und N-Bromsuccinimid im Molverhältnis 1:(1,5 bis 2,5), insbesondere 1:(1,8 bis 2,3), bevorzugt 1:(1,9 bis 2,2) ein.

Im Verlauf der Bromierung entsteht aus N- Bromsuccinimid das Succinimid, das, gegebenenfalls nach Abkühlen der das Reaktionsprodukt enthaltenden Lösung, durch Filtration abgetrennt wird. Eine andere Möglichkeit besteht darin, das gebildetete Succinimid durch Extraktion mit Wasser abzutrennen. Üblicherweise setzt man hierfür 10 bis 100 Gew.-% Wasser, bezogen auf Reaktionsgemisch, ein.

Eine besonders einfache und zugleich wirksame Methode, Succinimid zu entfernen, besteht darin, das gebildete Succinimid in einem ersten Schritt durch Filtration und in einem zweiten Schritt durch Extraktion mit Wasser aus dem Reaktionsgemisch abzutrennen. Hierbei setzt man vergleichsweise wenig Wasser ein und erhält auch dementsprechend wenig Abwasser.

Die unter Einfluß von Licht durchgeführte Bromierung liefert das gewünschte Wertprodukt in einer Ausbeute von etwa 80 %. Daneben entstehen üblicherweise 1 bis 6 % eines bromierten Stoffes, vermutlich 2-Brommethyl-2'-methyl-1,1'-binaphthyl, und 7 bis 13 % eines weiteren Nebenproduktes, vermutlich 2-Dibrommethyl-2'-brommethyl-1,1'-binaphthyl. Das entsprechende, in die Arbusov-Reaktion eingesetzte Reaktionsgemisch (A) enthält neben geringen Mengen nicht näher identifizierter Stoffe vermutlich die vorstehend genannten Bromverbindungen gelöst im Lösungsmittel, beispielsweise in Chlorbenzol und/oder Dichlorbenzol, insbesondere in Chlorbenzol.

Anstelle des unter Einwirkung von Licht hergestellten Reaktionsgemisches (A) läßt sich auch ein Reaktionsgemisch (B), das beispielsweise durch Umsetzung von 2,2'-Dimethyl-1,1'-binaphthyl mit N-Bromsuccinimid in Anwesenheit eines Lösungsmittel unter Einwirkung eines Radikalbildners (Radikalstarter) und durch nachfolgende Abtrennung von Succinimid erhältlich ist, mit gutem Erfolg in die Arbusov-Reaktion (Umsetzung mit dem Diphenylphosphinigsäurealkylester) einsetzen. Man kann ein solches Reaktionsgemisch (B) herstellen, indem man 2,2'-Dimethyl-1,1'-binaphthyl mit N-Bromsuccinimid, beispielsweise im Molverhältnis 1:(1,5 bis 2,5), insbesondere 1:(1,8 bis 2,3), bevorzugt 1:(1,9 bis 2,2) bei erhöhter Temperatur, üblicherweise bei Temperaturen von 25 bis 150, insbesondere 40 bis 135 °C, unter Einwirkung eines Radikalstarters in Chlorbenzol und/oder Dichlorbenzol umsetzt, gebildetes Succinimid abtrennt, ohne jedoch das als Lösungsmittel verwendete Chlorbenzol und/oder Dichlorbenzol abzutrennen.
Als Radikalstarter eignen sich die üblichen Radikalbildner z.B. organische Peroxide, Hydroperoxide, Azo-bis-isobutyronitril.

Die unter Einwirkung des Radikalstarters durchgeführte Bromierung liefert das gewünschte Wertprodukt in einer Ausbeute von etwa 63 bis 67 %. Daneben entstehen in der Regel 10 bis 20 % eines bromierten Produktes, vermutlich 2-Brommethyl-2'-methyl-1,1'-binaphthyl und 8 bis 15 % eines weiteren Nebenproduktes, vermutlich 2-Dibrommethyl-2-brommethyl-1,1'-binaphthyl. Das in die Arbusov-Reaktion eingesetzte Reaktionsgemisch (B) enthält neben geringen Mengen nicht näher identifizierter Stoffe vermutlich die vorstehend genannten Bromverbindungen gelöst in Chlorbenzol und/oder Dichlorbenzol, insbesondere Chlorbenzol.

Man trennt aus dem Bromierungsgemisch gegebenenfalls nach Abkühlen, das aus dem N-Bromsuccinimid gebildete Succinimid durch Filtration ab. Eine andere Möglichkeit besteht darin, daß man die nachfolgende Abtrennung von Succinimid durch Extraktion mit Wasser durchführt.
Eine besonders einfache und wirksame Variante besteht darin, daß man die Abtrennung von Succinimid aus dem Bromierungsgemisch in einem ersten Schritt durch Filtration und in einem zweiten Schritt durch Extraktion mit Wasser durchführt.

Nichtumgesetzten Radikalbildner kann man, beispielsweise durch Wäsche mit einer wäßrigen Na₂SO₃-Lösung, entfernen.

Man erhält auf die vorstehend beschriebene Weise das Reaktionsgemisch (B).

Zur Durchführung der Arbusov-Reaktion setzt man das Reaktionsgemisch, beispielsweise das Reaktionsgemisch (A) oder das Reaktionsgemisch (B), mit einem Diphenylphosphinigsäurealkylester (Formel IV), wie zuvor bereits erwähnt, bei 70 bis 180 °C um. In einer Vielzahl von Fällen genügt es die Umsetzung bei 100 bis 170, insbesondere 120 bis 160 °C durchzuführen. Die Umsetzung verläuft nach folgender Gleichung:

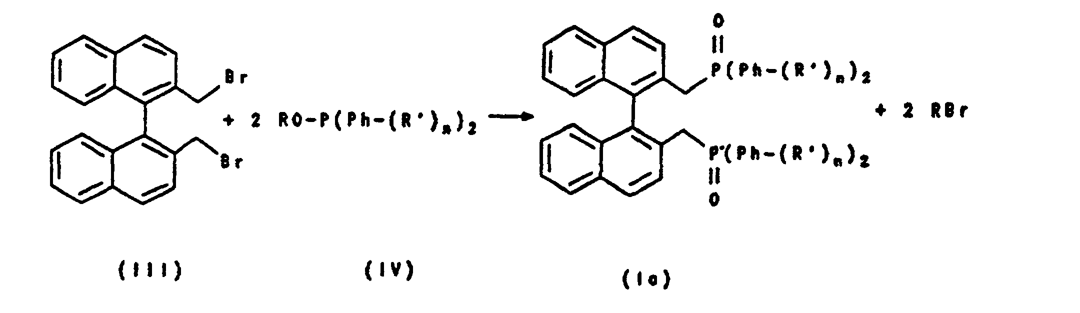

Üblicherweise setzt man als Diphenylphosphinigsäurealkylester der Formel (IV) einen Diphenylphospinigsäure(C₁-C₅)alkylester oder Mischungen dieser Ester ein. Geeignet als Diphenylphosphinigsäurealkylester sind, ohne Anspruch auf Vollständigkeit zu erheben, Diphenylphosphinigsäuremethylester, Diphenylphosphinigsäureethylester, Diphenylphosphinigsäureisopropylester, Diphenylphosphinigsäure-n-propylester, Bis-(3-fluorphenyl)-phosphinigsäureethylester, Bis-(4-fluorphenyl)phosphinigsäurethylester, Bis-(2-methylphenyl)phosphinigsäureethylester und/oder Bis-(3-trifluormethylphenyl)-phosphinigsäureethylester.

Im allgemeinen setzt man den Diphenylphosphinigsäurealkylester, bezogen auf das 2,2'-Dimethyl-1,1'-binaphthyl, im Molverhältnis (1,8 bis 4):1, insbesondere (1,9 bis 3):1, bevorzugt (2 bis 2,5):1 ein. Man kann jedoch den Diphenylphosphinigsäurealkylester (Formel IV), bezogen auf eingesetztes 2,2'-Dimethyl-1,1'-binaphthyl, für eine Vielzahl von Fällen in stöchiometrischem Verhältnis oder in geringem Überschuß einsetzen.

Bevor man das aus der Stufe der Bromierung resultierende Reaktionsgemisch in die nachfolgende Arbusov-Reaktion (Umsetzung mit dem Diphenylphosphinigsäurealkylester) einsetzt, empfiehlt es sich, wie zuvor erwähnt, in einer Reihe von Fällen, das nichtumgesetzte Bromierungsmittel und/oder das umgesetzte Bromierungsmittel und/oder das in der Stufe der Bromierung verwendete Lösungsmittel abzutrennen.
Nichtumgesetztes Bromierungsmittel, beispielsweise Brom, läßt sich durch Verdampfen entfernen, umgesetztes Bromierungsmittel, beispielsweise Succinimid, durch Filtration und Extraktion. Das in der Stufe der Bromierung verwendete Lösungsmittel wird durch ein weiteres Lösungsmittel, das höher als das ursprünglich eingesetzte Lösungsmittel siedet, ersetzt. Man setzt das weitere Lösungsmittel vor der Umsetzung oder während der Umsetzung oder nach der Umsetzung des Reaktionsgemisches mit dem Diphenylphosphinigsäurealkylester zu und destilliert anschließend das ursprünglich eingesetzte Lösungsmittel ab. Diese Abtrennung des ursprünglichen Lösungsmittel kann vor der Arbusov-Reaktion oder auch während der Arbusov-Reaktion oder nach der Arbusov-Reaktion erfolgen. Verwendet man ein gegenüber der Arbusov-Reaktion inertes Lösungsmittel als ursprüngliches Lösungsmittel, so kann man die Abtrennung während der Arbusov-Reaktion durchführen oder auch auf die Abtrennung verzichten. Derartige Lösungsmittel sind beispielsweise Chlorbenzol, Dichlorbenzol und Gemische derselben, bei deren Gebrauch eine Abtrennung nicht unbedingt erforderlich ist.
Lösungsmittel, die gegenüber der Arbusov-Reaktion nicht inert sind, sind vor Durchführung der Arbusov-Reaktion zu entfernen. Derartige Lösungsmittel sind beispielsweise einfach oder mehrfach chlorierte aliphatische Kohlenwasserstoffe.
Als weiteres Lösungsmittel eignen sich generell höher als das ursprünglich eingesetzte Lösungsmittel siedende Lösungsmittel, die unter den Bedingungen der Arbusov-Reaktion inert sind. Hierunter fallen aromatische Kohlenwasserstoffe, beispielsweise Toluol, o-Xylol, m-Xylol, p-Xylol, Gemische dieser Xylole, Ethylbenzol und/oder Mesitylen und hochsiedende aliphatische Kohlenwasserstoffe, beispielsweise Petrolether mit einem Siedepunkt > 100 °C, Decalin, Ligroin und/oder Isooctan.
Die Arbusov-Reaktion läßt sich somit in Anwesenheit oder Abwesenheit eines weiteren Lösungsmittels durchführen.

Es hat sich in einigen Fällen als vorteilhaft erwiesen, das weitere, unpolare Lösungsmittel, das höher als das ursprünglich eingesetzte Lösungsmittel siedet, während der Arbusov-Reaktion (Umsetzung mit dem Diphenylphosphinigsäurealkylester) zuzusetzen und gleichzeitig das ursprünglich eingesetzte Lösungmittel, beispielsweise Chlorbenzol und/oder Dichlorbenzol abzudestillieren. Als Lösungsmittel sind hierfür beispielsweise höher als Chlorbenzol und/oder Dichlorbenzol siedende aromatische Verbindungen, beispielsweise o-Xylol, m-Xylol, p-Xylol, Gemische dieser Xylole und/oder Mesitylen, geeignet. Dadurch läßt sich die Ausbeute an isoliertem 2,2'-Bis-(diphenylphosphinylmethyl)-1,1'-binaphthyl nochmals etwas steigern.

Es ist als überraschend anzusehen, daß generell die bromierten Binaphthyle, beispielsweise die sowohl in der Reaktionsmischung (A) als auch in der Reaktionsmischung (B) enthaltenen bromierten Nebenprodukte, zu keiner Störung in der Arbusov-Reaktion führen und auch die Qualität des gewünschten Endproduktes (Formel I) nicht beeinträchtigen. Ferner war nicht zu erwarten, daß die Verwendung eines Lösungsmittels, beispielsweise der Gebrauch von Chlorbenzol und/oder Dichlorbenzol in der Arbusov-Reaktion zu einer signifikanten Steigerung der Ausbeute führt. Während das Verfahren der JP 7 939 059 eine Ausbeute von lediglich 30 % ergibt, liefert das erfindungsgemäße Verfahren eine Ausbeute von 90 bis 95 % an gewünschtem Wertprodukt, jeweils bezogen auf 2,2'-Bis(brommethyl)-1,1'-binaphthyl. Zudem wird auch die Gefahr eines explosionsartigen Verlaufs der Umsetzung vermieden.

Zur Durchführung der Arbusov-Reaktion legt man üblicherweise das Reaktionsgemisch, beispielsweise Reaktionsgemisch (A) oder das Reaktionsgemisch (B) oder Mischungen der beiden Reaktionsgemische, vor und erwärmt auf die vorgegebene Temperatur. Es ist vorteilhaft, eine Temperatur unterhalb des Siedepunktes des verwendeten Lösungsmittels zu wählen. Nach Erreichen der Reaktionstemperatur tropft man den Diphenylphosphinigsäurealkylester langsam zu der vorgelegten Reaktionslösung zu. Infolge der ablaufenden Umsetzung bildet sich Alkylbromid, das kontinuierlich aus dem Reaktionsprodukt abdestilliert wird. Falls gewünscht, kann man nach Beendigung der Zugabe des Diphenylphosphinigsäurealkylesters auch einige Zeit am Rückfluß kochen, um die Umsetzung zu vervollständigen.

Beabsichtigt man, das ursprünglich eingesetzte Lösungsmittel während der Arbusov-Reaktion zu entfernen, so wählt man eine Temperatur oberhalb der Siedetemperatur des ursprünglichen Lösungsmittels, aber unterhalb der zugesetzten weiteren Lösungsmittel. Auf diese Weise destilliert sowohl das Alkylbromid als auch das ursprünglich eingesetzte Lösungsmittel ab.

Das erfindungsgemäße Verfahren macht durch Umsetzung von 2,2'-Bis(brommethyl)-1,1'-binaphthyl der Formel (III) mit dem entsprechenden Diphenylphosphinigsäureester der Formel IV die neuen Verbindungen der Formel (Ia) 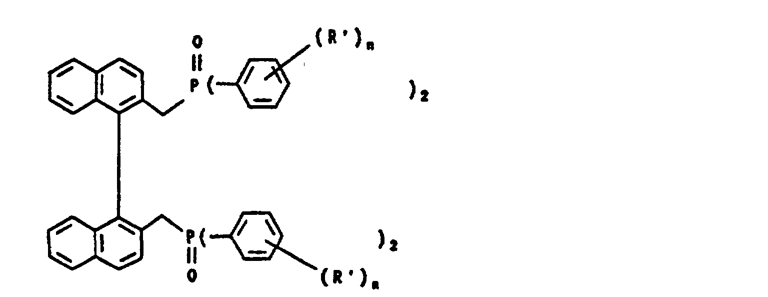 worin R' für einen Alkylrest mit 1 bis 4 Kohlenstoffatomen, CF₃, Fluor, Chlor oder Brom, insbesondere für Methyl, CF₃ oder Fluor, und n für 1 oder 2 steht, auf überraschend einfache Weise zugänglich.

Als neue Verbindungen, die hierunter fallen, sind besonders
2,2'-Bis-[bis(3-fluorphenyl)phosphinylmethyl]-1,1'-binaphthyl,
2,2'-Bis-[bis(4-fluorphenyl)phosphinylmethyl]-1,1'-binaphthyl,
2,2'-Bis-[bis(2-methylphenyl)phosphinylmethyl]-1,1'-binaphthyl und
2,2'-Bis-[bis(3-trifluormethyl)phenylphosphinylmethyl]-1,1'-binaphthyl zu erwähnen.

Die nachfolgenden Beispiele belegen die Erfindung, ohne sie zu beschränken.

### Experimenteller Teil:

### Beispiel 1

### Herstellung unter Verwendung eines Reaktionsgemisches A

In einem 4 l-Glaskolben werden unter Feuchtigkeitsausschluß 282,4 g (1,0 mol) 2,2'-Dimethyl-1,1'-binaphthyl und 373,8 g (2,1 mol) N-Bromsuccinimid in 1,7 I Chlorbenzol suspendiert und 8 Stunden bei 5 bis 10 °C mit einer UV-Tauchlampe belichtet. Man filtriert das ausgefallene Succinimid ab, extrahiert zweimal mit je 200 ml Wasser, trocknet mit Natriumsulfat und filtriert. Das Filtrat (Reaktionsgemisch A)* wird in einen 4 I-Vierhalskolben mit Rührer, Tropftrichter Rückflußkühler und Innenthermometer überführt und auf 125 °C erwärmt. Danach tropft man 460,5 g (2,0 mol) Diphenylphosphinig-säureethylester langsam zu, wobei Ethylbromid abdestilliert. Nach dem Ende der Zugabe erhitzt man noch zwei Stunden zum Rückfluß und kühlt dann auf 0 °C. ab. Man filtriert den Feststoff ab, wäscht mit kaltem Chlorbenzol und trocknet i. Vak. Man erhält 512 g (Reinheit: > 99 %) 2,2'-Bis-(diphenylphosphinylmethyl)-1,1'-binaphthyl als farblose Kristalle mit Schmp. 287 bis 289 °C, entsprechend 75 % Gesamtausbeute bezogen auf eingesetztes 2,2'-Dimethyl-1,1'-binaphthyl.

* Zusammensetzung nach GC-Analyse (in Mol%) ohne Lösungsmittel:
80 % 2,2'-Bis(brommethyl)-1,1'-binaphthyl
2 % 2-Brommethyl-2'-methyl-1,1'-binaphthyl
13 % 2-Dibrommethyl-2'-brommethyl-1,1'-binaphthyl.

### Beispiel 2

### Herstellung unter Verwendung eines Reaktionsgemisches A

In einem 4 I-Glaskolben werden unter Feuchtigkeitsausschluß 282,4 g (1,0 mol) 2,2'-Dimethyl-1,1'-binaphthyl und 373,8 g (2,1 mol) N-Bromsuccinimid in 1,7 Chlorbenzol suspendiert und 8 Stunden bei 5 bis 10 °C mit einer UV-Tauchlampe belichtet. Man filtriert das ausgefallene Succinimid ab, extrahiert zweimal mit je 200 ml Wasser, trocknet mit Natriumsulfat und filtriert. Das Filtrat (Reaktionsgemisch A)* wird in einen 4 I-Vierhalskolben mit Rührer, Tropftrichter Rückflußkühler und Innenthermometer überführt und auf 125 °C erwärmt. Danach tropft man 488,6 g (2,0 mol) Diphenylphosphinigsäurepropylester langsam zu, wobei n-Propylbromid abdestilliert. Nach dem Ende der Zugabe erhitzt man noch zwei Stunden zum Rückfluß und kühlt dann auf 0 °C ab. Man filtriert den Feststoff ab, wäscht mit kaltem Chlorbenzol und trocknet im Vakuum. Man erhält 502,5 g 2,2'-Bis(diphenylphosphinylmethyl)-1,1'-binaphthyl (Reinheit > 99 %) als farblose Kristalle mit Schmp. 287 bis 289 °C, entsprechend 73,5 % Gesamtausbeute, bezogen auf eingesetztes 2,2'-Dimethyl-1,1'-binaphthyl.

* Zusammensetzung nach GC-Analyse (in Mol%) ohne Lösungsmittel
68 % 2,2'-bis(brommethyl)-1,1'-binaphthyl
15 % 2-Brommethyl-2'-methyl-1,1'-binaphthyl
13 % 2-Dibrommethyl-2'-brommethyl-1,1'-binaphthyl

### Beispiel 3

### Herstellung unter Verwendung eines Reaktionsgemisches B

In einem 4 I-Glaskolben werden unter Feuchtigkeitsauschluß 282,4 g (1.0 mol) 2,2'-Dimethyl-1,1'-binaphthyl, 373,8 g (2,1 mol) N-Bromsuccinimid und 500 mg Benzoylperoxid in 1,7 I Chlorbenzol suspendiert und 3,5 Stunden bei Siedetemperatur gerührt. Man filtriert das ausgefallene Succinimid ab, extrahiert zweimal mit je 200 ml Wasser und einmal mit 100 ml Na₂SO₃ Lösung, trocknet mit Natriumsulfat und filtriert. Das Filtrat (Reaktionsgemisch B)* wird in einen 4 I-Vierhalskolben mit Rührer, Tropftrichter, Rückflußkühler und Innenthermometer überführt und auf 125 °C erwärmt. Danach tropft man 460,5 g (2.0 mol) Diphenylphosphinig-säureethylester langsam zu, wobei Ethylbromid abdestilliert. Nach dem Ende der Zugabe erhitzt man noch zwei Stunden zum Rückfluß und kühlt dann auf 0 °C ab. Man filtriert den Feststoff ab, wäscht mit kaltem Chlorbenzol und trocknet im Vakuum. Man erhält 443,8 g (Reinheit > 99 %) 2,2'-Bis(diphenylphosphinylmethyl)-1,1'-binaphthyl als farblose Kristalle mit Schmp. 287 bis 289 °C, entsprechend 65 % Gesamtausbeute, bezogen auf eingesetztes 2,2'-Dimethyl-1,1'-binaphthyl.

* Zusammensetzung nach GC-Analyse (in Mol%) ohne Lösungsmittel
68 % 2,2'-Bis(brommethyl)-1,1'-binaphthyl
15 % 2-Brommethyl-2'-methyl-1,1'-binaphthyl
13 % 2-Dibrommethyl-2'-brommethyl-1,1'-binaphthyl.

### Beispiel 4

### 2,2'-Bis-[bis(3-fluorphenyl)phosphinylmethyl]-1,1'-binaphthyl

Unter Rühren und Stickstoff tropft man 8,8 g (33 mmol) Bis-(3-fluorphenyl)phosphinigsäureethylester langsam zu einer auf 120°C erhitzten Lösung von 7,27 g (16,6 mmol) 2,2'-Bis(brommethyl)-1,1'-binaphthyl in 40 ml o-Xylol, wobei Ethylbromid abdestilliert. Nach dem Ende der Zugabe erhitzt man noch zwei Stunden zum Rückfluß und kühlt dann auf 0°C ab. Man filtriert den Feststoff ab, wäscht mit kaltem o-Xylol und trocknet i. Vak. Man erhält 11,5 g (91 %) farblose Kristalle mit Schmp. 280-284°C.
³¹P-NMR: δ (CDCl₃) = 27,3 ppm

### Beispiel 5:

### 2, 2'-Bis-[bis(4-fluorphenyl)phosphinylmethyl]-1,1'-binaphthyl

Unter Rühren und Stickstoff werden zu einer auf 135°C erwärmten Lösung von 8,80 g (0,02 mol) 2,2'-Bis(brommethyl)-1,1'-binaphthyl in 40 ml o-Xylol 10,70 g (0,04 mol) Bis(4-fluorphenyl)phosphinigsäureethylester getropft. Nach dem Ende der Zugabe wird noch 1,5 Stunden zum Rückfluß erhitzt. Nach dem Abkühlen auf Raumtemperatur werden die ausgefallenen Kristalle abgesaugt und mit Xylol gewaschen. Man erhält 14,1 g (94 %) farblose Kristalle mit Schmp. 211-214°C.
³¹P-NMR: δ (CDCl₃) = 28,2 ppm

### Beispiel 6

### 2,2'-Bis-[bis(2-methylphenyl)phosphinylmethyl]-1,1'-binaphthyl

Unter Rühren und Stickstoff tropft man 21,5 g (83 mmol) Bis-(2-methylphenyl)phosphinigsäureethylester langsam zu einer auf 120°C erhitzten Lösung von 18,4 g (42 mmol) 2,2'-Bis(brommethyl)-1,1'-binaphthyl in 100 ml o-Xylol, wobei Ethylbromid abdestilliert. Nach dem Ende der Zugabe erhitzt man noch zwei Stunden zum Rückfluß und kühlt dann auf 0°C ab. Man filtriert den Feststoff ab, wäscht mit kaltem o-Xylol und trocknet i. Vak. Man erhält 25,1 g (81 %) farblose Kristalle mit Schmp. 231-234°C.
³¹P-NMR: δ (CDCl₃) = 30,5 ppm

### Beispiel 7

### 2,2'-Bis-[bis-(3-trifluormethylphenyl)phosphinylmethyl]-1,1'-binaphthyl

Unter Rühren und Stickstoff tropft man 20,1 g (55 mmol) Bis-(3-trifluormethylphenyl)phosphinigsäureethylester langsam zu einer auf 120°C erhitzten Lösung von 11,0 g (25 mmol) 2,2'-Bis(brommethyl)-1,1'-binaphthyl in 80 ml o-Xylol, wobei Ethylbromid abdestilliert. Nach dem Ende der Zugabe erhitzt man noch zwei Stunden zum Rückfluß und kühlt dann auf 0°C ab. Man filtriert den Feststoff ab, wäscht mit kaltem o-Xylol und trocknet i. Vak. Man erhält 16,1 g (68 %) farblose Kristalle.
³¹P-NMR: δ (CDCl₃) = 27,1 ppm

### Beispiel 8 (Arbusov in Ethylacetat)

Unter Rühren und Stickstoff tropft man 88,5 g (385 mmol)
Diphenylphosphinigsäureethylester langsam zu einer auf 78°C erhitzten Lösung von 77,0 g (175 mmol) 2,2'-Bis(brommethyl)-1,1'-binaphthyl in 400 ml Ethylacetat und destilliert gleichzeitig Ethylbromid über eine 80 cm-Vigreuxkolonne ab. Nach dem Ende der Zugabe erhitzt man noch zwei Stunden zum Rückfluß und kühlt dann auf 0°C ab. Man filtriert den Feststoff ab, wäscht mit kaltem Ethylacetat und trocknet i. Vak. Man erhält 85,0 g (71 %) farblose Kristalle mit Schmp. 287-289°C.

## Patentansprüche

1. Verfahren zur Herstellung von 2,2'-Bis(diphenylphosphinylmethyl)-1,1'-binaphthylen, dadurch gekennzeichnet, daß man ein in einem Lösungsmittel durch Umsetzung von 2,2'-Dimethyl-1,1'-binaphthyl mit einem Bromierungsmittel hergestelltes, 2,2'-Bis(brommethyl)-1,1'-binaphthyl und weitere bromierte Binaphthyle enthaltendes Reaktionsgemisch, gegebenenfalls nach Abtrennung von nichtumgesetztem Bromierungsmittel, umgesetztem Bromierungsmittel und/oder Lösungsmittel, mit einem Diphenylphosphinigsäurealkylester der Formel RO-P(Ph-R')ₙ)₂, worin R für einen Alkylrest mit 1 bis 5 Kohlenstoffatomen, Ph für Phenyl, R' für einen Alkylrest mit 1 bis 4 Kohlenstoffatomen, CF₃, Fluor, Chlor oder Brom und n für 0, 1 oder 2 steht bei 70 bis 180°C, gegebenenfalls in Anwesenheit eines weiteren Lösungsmittels, umsetzt.

2. Verfahren nach Anspruch 1, dadurch gekennzeichnet, daß man 2,2'-Dimethyl-1,1'-binaphthyl und das Lösungsmittel im Gewichtsverhältnis 1 : (3 bis 40) einsetzt.

3. Verfahren nach Anspruch 2, dadurch gekennzeichnet, daß man 2,2'-Dimethyl-1,1'-binaphthyl und das Lösungsmittel im Gewichtsverhältnis 1 : (4 bis 20) einsetzt.

4. Verfahren nach Anspruch 3, dadurch gekennzeichnet, daß man 2,2'-Dimethyl-1,1'-binaphthyl und das Lösungsmittel im Gewichtsverhältnis 1: (5 bis 15) einsetzt.

5. Verfahren nach einem oder mehreren der Ansprüche 1 bis 4, dadurch gekennzeichnet, daß man als Lösungsmittel ein einfach oder mehrfach chloriertes Benzol, einen einfach oder mehrfach chlorierten aliphatischen Kohlenwasserstoff, einen Ester einer aliphatischen Carbonsäure mit 1 bis 6 Kohlenstoffatomen und eines Alkylalkohols mit 1 bis 4 Kohlenstoffatomen oder Mischungen derselben einsetzt.

6. Verfahren nach einem oder mehreren der Ansprüche 1 bis 5, dadurch gekennzeichnet, daß man als Lösungsmittel Chlorbenzol oder Dichlorbenzol einsetzt.

7. Verfahren nach einem oder mehreren der Ansprüche 1 bis 6, dadurch gekennzeichnet, daß man als Lösungsmittel eine Mischung aus Chlorbenzol und Dichlorbenzol einsetzt.

8. Verfahren nach einem oder mehreren der Ansprüche 1 bis 7, dadurch gekennzeichnet, daß man 2,2'-Dimethyl-1,1'-binaphthyl und das Bromierungsmittel unter Einwirkung von Licht einer Wellenlänge von 10⁻⁵ bis 10⁻⁸ m bei-10 bis 75°C umsetzt.

9. Verfahren nach Anspruch 8, dadurch gekennzeichnet, daß man 2,2'-Dimethyl-1,1'-binaphthyl und das Bromierungsmittel unter Einwirkung von Licht einer Wellenlänge von 10⁻⁵ bis 10⁻⁸ m bei -5 bis +50°C umsetzt.

10. Verfahren nach Anspruch 9, dadurch ghekennzeichnet, daß man 2,2'-Dimethyl-1,1'-binaphthyl und das Bromierungsmittel unter Einwirkung von Licht einer Wellenlänge von 10⁻⁵ bis 10⁻⁸ m bei 0 bis 40°C umsetzt.

11. Verfahren nach einem oder mehreren der Ansprüche 1 bis 10, dadurch gekennzeichnet, daß man 2,2'-Dimethyl-1,1'-binaphthyl und das Bromierungsmittel unter Einwirkung eines Radikalbildners bei 25 bis 150°C umsetzt.

12. Verfahren nach Anspruch 11, dadurch gekennzeichnet, daß man 2,2'-Dimethyl-1,1'-binaphthyl und das Bromierungsmittel unter Einwirkung eines Radikalbildners bei 40 bis 135°C umsetzt.

13. Verfahren nach einem oder mehreren der Ansprüche 1 bis 12, dadurch gekennzeichnet, daß man 2,2'-Dimethyl-1,1'-binaphthyl mit N-Bromsuccinimid als Bromierungsmittel umsetzt.

14. Verfahren nach einem oder mehreren der Ansprüche 1 bis 13, dadurch gekennzeichnet, daß man 2,2'-Dimethyl-1,1'-binaphthyl und N-Bromsuccinimid im Molverhältnis 1 : (1,5 bis 2,5) einsetzt.

15. Verfahren nach Anspruch 14, dadurch gekennzeichnet, daß man 2,2'-Dimethyl-1,1'-binaphthyl und N-Bromsuccinimid im Molverhältnis 1: (1,8 bis 2,3) einsetzt.

16. Verfahren nach Anspruch 15, dadurch gekennzeichnet, daß man 2,2'-Dimethyl-1,1'-binaphthyl und N-Bromsuccinimid im Molverhältnis 1 : (1,9 bis 2,2) einsetzt.

17. Verfahren nach einem oder mehreren der Ansprüche 8 bis 16, dadurch gekennzeichnet, daß man nach Bromierung das als umgesetztes Bromierungsmittel gebildete Succinimid abtrennt.

18. Verfahren nach einem oder mehreren der Ansprüche 8 bis 17, dadurch gekennzeichnet, daß man Succinimid, gegebenenfalls nach Abkühlen, durch Filtration abtrennt.

19. Verfahren nach einem oder mehreren der Ansprüche 8 bis 18, dadurch gekennzeichnet, daß man Succinimid durch Extraktion mit Wasser abtrennt.

20. Verfahren nach einem oder mehreren der Ansprüche 8 bis 18, dadurch gekennzeichnet, daß man Succinimid in einem ersten Schritt durch Filtration und in einem zweiten Schritt durch Extraktion mit Wasser abtrennt.

21. Verfahren nach einem oder mehreren der Ansprüche 1 bis 20, daudrch gekennzeichnet, daß man das Reaktionsgemisch mit dem Diphenylphosphinigsäurealkylester der Formel RO-P(Ph-(R')ₙ)₂ bei 100 bis 170°C umsetzt.

22. Verfahren nach Anspruch 21, dadurch gekennzeichnet, daß man das Reaktionsgemisch mit dem Diphenylphosphinigsäurealkylester der Formel RO-P(Ph-(R')ₙ)₂ bei 120 bis 160°C umsetzt

23. Verfahren nach einem oder mehreren der Ansprüche 1 bis 22, dadurch gekennzeichnet, daß man den Diphenylphosphinigsäurealkylester der Formel RO-P(Ph-(R'ₙ)₂, bezogen auf eingesetztes 2,2'-Dimethyl-1,1'-binaphthyl, im Molverhältnis (1,8 bis 4) : 1 einsetzt.

24. Verfahren nach Anspruch 23, dadurch gekennzeichnet, daß man den Diphenylphosphinigsäurealkylester der Formel RO-P(Ph-(R'ₙ)₂, bezogen auf eingesetztes 2,2'-Dimethyl-1,1'-binaphthyl, im Molverhältnis (1,9 bis 3) : 1 einsetzt.

25. Verfahren nach Anspruch 24, dadurch gekennzeichnet, daß man den Diphenylphosphinigsäurealkylester der Formel RO-P(Ph-(R'ₙ)₂, bezogen auf eingesetztes 2,2'-Dimethyl-1,1'-binaphthyl, im Molverhältnis (2 bis 2,5) : 1 einsetzt.

26. Verfahren nach einem oder mehreren der Ansprüche 1 bis 25, dadurch gekennzeichnet, daß man als Diphenylphosphinigsäurealkylester der Formel RO-P(Ph-(R'ₙ)₂ einen Diphenylphosphingsäure(C₁-C₅)alkylester oder Mischungen dieser Ester einsetzt.

27. Verfahren nach einem oder mehreren der Ansprüche 1 bis 26, dadurch gekennzeichnet, daß man als Diphenylphosphinigsäurealkylester der Formel RO-P(Ph-(R'ₙ)₂ den Diphenylphosphinigsäuremethylester, Diphenylphosphinigsäureethylester, Diphenylphosphinigsäureisopropylester, Diphenylphosphinigsäure-n-propylester, Bis-(3-fluorpheny)-phosphinigsäureethylester, Bis-(4-fluorphenyl)phosphinigsäureethylester, Bis-(2-methylphenyl)phosphinigsäureethylester und/oder Bis-(3-trifluormethylphenyl)phosphinigsäureethylester) einsetzt.

28. Verfahren nach einem oder mehreren der Ansprüche 1 bis 27, dadurch gekennzeichnet, daß man vor der Umsetzung oder während der Umsetzung oder nach der Umsetzung des Reaktionsgemisches mit dem Diphenylphosphinigsäurealkylester ein weiteres Lösungsmittel, das höher als das ursprünglich eingesetzte Lösungsmittel siedet, zusetzt und das ursprünglich eingesetzte Lösungsmittel abdestilliert.

29. Verfahren nach einem oder mehreren der Ansprüche 1 bis 28, dadurch gekennzeichnet, daß man während der Umsetzung des Reaktionsgemisches mit dem Diphenylphosphinigsäurealkylester als weiteres Lösungsmittel o-Xylol, m-Xylol, p-Xylol, Gemische dieser Xylole und/oder Mesitylen zusetzt und Chlorbenzol und/oder Dichlorbenzol als ursprünglich eingeseztes Lösungsmittel abdestilliert.

30. Verbindungen der Formel 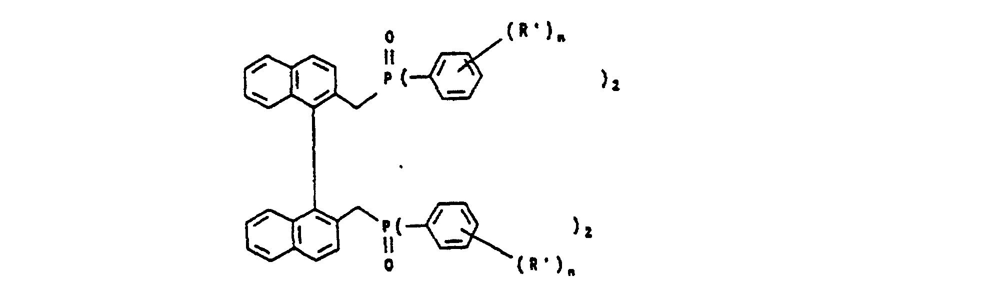 worin R' für einen Alkylrest mit 1 bis 4 Kohlenstoffatomen, CF₃, Fluor, Chlor oder Brom und n für 1 oder 2 steht.

31. Verbindungen der Formel 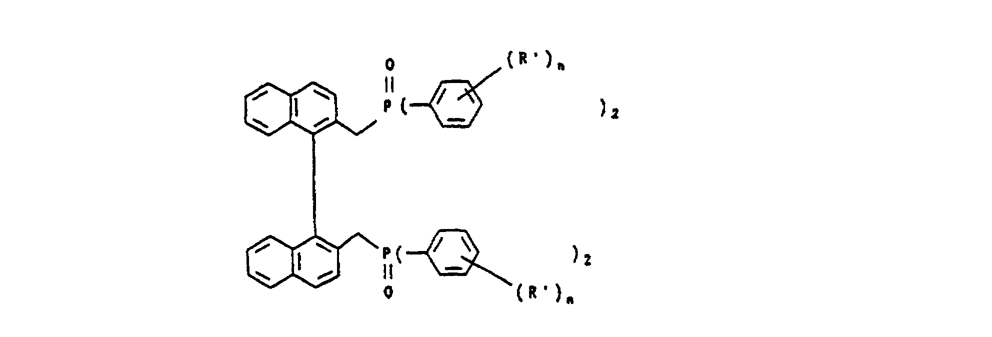 worin R' für Methyl, CF₃ oder Fluor und n für 1 oder 2 steht.

32. Die Verbindungen 2,2'-Bis-[bis(3-fluorphenyl)phosphinylmethyl]-1,1'-binaphthyl, 2,2'-Bis-[bis(4-fluorphenyl)phosphinylmethyl]-1,1'-binaphthyl, 2,2'-Bis-[bis(2-methylphenyl)phosphinylmethyl]-1,1'-binaphthyl und 2,2'-Bis-[bis(3-trifluormethylphenyl)phosphinylmethyl]-1,1'-binaphthyl.

## Claims

1. A process for preparing 2,2'-bis(diphenylphosphinomethyl)-1,1'-binaphthyls, which comprises reacting a reaction mixture prepared in a solvent by reaction of 2,2'-dimethyl-1,1'-binaphthyl with a brominating agent and containing 2,2'-bis(bromomethyl)-1,1'-binaphthyl and further brominated binaphthyls, if desired after removal of unreacted brominating agent, reacted brominating agent and/or solvent, with an alkyl diphenylphosphinite of the formula RO-P(Ph-(R')ₙ)₂, where R is an alkyl radical having from 1 to 5 carbon atoms, Ph is phenyl, R' is an alkyl radical having from 1 to 4 carbon atoms, CF₃, fluorine, chlorine or bromine and n is 0, 1 or 2, at from 70 to 180°C in the presence of absence of an further solvent.

2. The process as claimed in claim 1, wherein 2,2'-dimethyl-1,1-binaphthyl and the solvent are used in a weight ratio of 1:(3 to 40).

3. The process as claimed in claim 2, wherein 2,2'-dimethyl-1,1-binaphthyl and the solvent are used in a weight ratio of 1:(4 to 20).

4. The process as claimed in claim 3, wherein 2,2'-dimethyl-1,1-binaphthyl and the solvent are used in a weight ratio of 1:(5 to 15).

5. The process as claimed in one or more of claims 1 to 4, wherein the solvent used is a monochlorinated or polychlorinated benzene, a monochlorinated or polychlorinated aliphatic hydrocarbon, an ester of an aliphatic carboxylic acid having from 1 to 6 carbon atoms and an alkyl alcohol having from 1 to 4 carbon atoms or a mixture thereof.

6. The process as claimed in one or more of claims 1 to 5, wherein the solvent used is chlorobenzene or dichlorobenzene.

7. The process as claimed in one or more of claims 1 to 6, wherein the solvent used is a mixture of chlorobenzene and dichlorobenzene.

8. The process as claimed in one or more of claims 1 to 7, wherein 2,2'-dimethyl-1,1'-binaphthyl and the brominating agent are reacted under the action of light having a wavelength of from 10⁻⁵ to 10⁻⁸ m at from -10 to 75°C.

9. The process as claimed in claim 8, wherein 2,2'-dimethyl-1,1'-binaphthyl and the brominating agent are reacted under the action of light having a wavelength of 10⁻⁵ to 10⁻⁸ m at from -5 to +50°C.

10. The process as claimed in claim 9, wherein 2,2'-dimethyl-1,1'-binaphthyl and the brominating agent are reacted under the action of light having a wavelength of 10⁻⁵ to 10⁻⁸ at from 0 to 40°C.

11. The process as claimed in one or more of claims 1 to 10, wherein 2,2'-dimethyl-1,1'-binaphthyl and the brominating agent are reacted under the action of a free-radical former at from 25 to 150°C.

12. The process as claimed in claim 11, wherein 2,2'-dimethyl-1,1'-binaphthyl and the brominating agent are reacted under the action of a free-radical former at from 40 to 135°C.

13. The process as claimed in one or more of claims 1 to 12, wherein 2,2'-dimethyl-1,1'-binaphthyl is reacted with N-bromosuccinimide as brominating agent.

14. The process as claimed in one or more of claims 1 to 13, wherein 2,2'-dimethyl-1,1'-binaphthyl and N-bromosuccinimide are used in a molar ratio of 1:(1.5 to 2.5).

15. The process as claimed in claim 14, wherein 2,2'-dimethyl-1,1'-binaphthyl and N-bromosuccinimide are used in a molar ratio of 1:(1.8 to 2.3).

16. The process as claimed in claim 15, wherein 2,2'-dimethyl-1,1'-binaphthyl and N-bromosuccinimide are used in a molar ratio of 1:(1.9 to 2.2).

17. The process as claimed in one or more of claims 8 to 16, wherein the succinimide formed as reacted brominating agent is removed after bromination.

18. The process as claimed in one or more of claims 8 to 17, wherein succinimide is removed, if desired after cooling, by filtration.

19. The process as claimed in one or more of claims 8 to 18, wherein succinimide is removed by extraction with water.

20. The process as claimed in one or more of claims 8 to 18, wherein succinimide is removed in a first step by filtration and in a second step by extraction with water.

21. The process as claimed in one or more of claims 1 to 20, wherein the reaction mixture is reacted with the alkyl diphenylphosphinite of the formula RO-P(Ph-(R')ₙ)₂ at from 100 to 170°C.

22. The process as claimed in claim 21, wherein the reaction mixture is reacted with the alkyl diphenylphosphinite of the formula RO-P(Ph-(R')ₙ)₂ at from 120 to 160°C.

23. The process as claimed in one or more of claims 1 to 22, wherein the alkyl diphenylphosphinite of the formula RO-P(Ph-(R')ₙ)₂ is used in a molar ratio of (1.8 to 4):1 based on 2,2-dimethyl-1,1'-binaphthyl used.

24. The process as claimed in claim 23, wherein the alkyl diphenylphosphinite of the formula RO-P(Ph-(R')ₙ)₂ is used in a molar ratio of (1.9 to 3):1 based on 2,2'-dimethyl-1,1'-binaphthyl used.

25. The process as claimed in claim 24, wherein the alkyl diphenylphosphinite of the formula RO-P(Ph-(R')ₙ)₂ is used in a molar ratio of (2 to 2.5):1 based on 2,2'-dimethyl-1,1'-binaphthyl used.

26. The process as claimed in one or more of claims 1 to 25, wherein the alkyl diphenylphosphinite of the formula RO-P(Ph-(R')ₙ)₂ which is used is a (C₁-C₅)-alkyl diphenylphosphinite or a mixture of these esters.

27. The process as claimed in one or more of claims 1 to 26, wherein the alkyl diphenylphosphinite of the formula RO-P(Ph-(R')ₙ)₂ which is used is methyl diphenylphosphinite, ethyl diphenylphosphinite, isopropyl diphenylphosphinite, n-propyl diphenylphosphinite, ethyl bis(3-fluorophenyl)phosphinite, ethyl bis(4-fluorophenyl)phosphinite, ethyl bis(2-methylphenyl)phosphinite and/or ethyl bis(3-tri-fluoromethylphenyl)-phosphinite).

28. The process as claimed in one or more of claims 1 to 27, wherein a further solvent which has a boiling point higher than that of the solvent originally used is added prior to the reaction or during the reaction or after the reaction of the reaction mixture with the alkyl diphenylphosphinite and the solvent originally used is distilled off.

29. The process as claimed in one or more of claims 1 to 28, wherein oxylene, m-xylene, p-xylene mixtures of these xylenes and/or mesitylene are added as a further solvent during the reaction of the reaction mixture with the alkyl diphenylphosphinite and chlorobenzene and/or dichlorobenzene are distilled off as the solvent originally used.

30. A compound of the formula 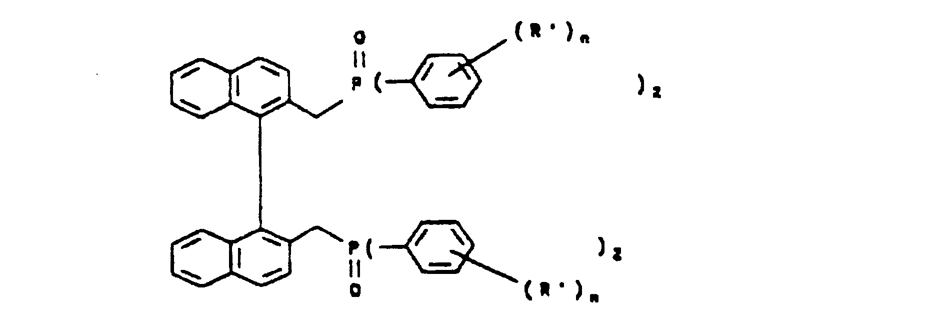 where R' is an alkyl radical having from 1 to 4 carbon atoms, CF₃, fluorine, chlorine or bromine and n is 1 or 2.

31. A compound of the formula 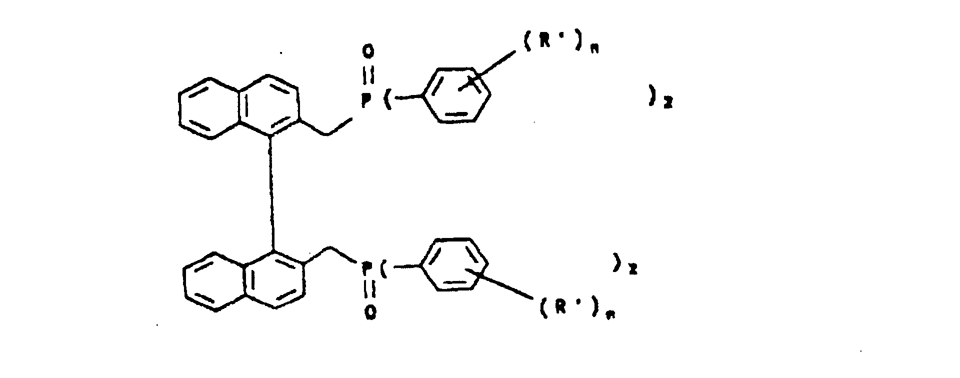 where R is methyl, CF₃ or fluorine and n is 1 or 2.

32. The compounds 2,2'-bis[bis(3-fluorophenyl)phosphinylmethyl]-1,1-binaphthyl, 2,2'-bis[bis(4-fluorophenyl)phosphinylmethyl]-1,1'-binaphthyl, 2,2'-bis[bis(2-methylphenyl)phosphinylmethyl]-1,1'-binaphthyl and 2,2'-bis[bis(3-trifluoromethylphenyl)-phosphinylmethyl]-1,1'-binaphthyl.

## Revendications

1. Procédé de préparation de 2,2'-bis(diphénylphosphinylméthyl)-1,1'-binaphtyles, caractérisé en ce que l'on fait réagir un mélange réactionnel préparé dans un solvant par réaction du 2,2'-diméthyl-1,1'-binaphtyle avec un agent bromant et contenant du 2,2'-bis(bromométhyl)-1,1'-binaphtyle et d'autres binaphtyles bromés, éventuellement après séparation de l'agent bromant qui n'a pas réagi, de l'agent bromant qui a réagi et/ou du solvant, avec un diphénylphosphinate d'alkyle de formule RO-P(Ph-(R')ₙ)₂, où R représente un reste alkyle ayant 1 à 5 atomes de carbone, Ph représente phényle, R' représente un reste alkyle ayant 1 à 4 atomes de carbone, CF₃, le fluor, le chlore ou le brome et n représente 0, 1 ou 2, à 70 à 180°C, éventuellement en présence d'un autre solvant.

2. Procédé selon la revendication 1, caractérisé en ce que l'on utilise le 2,2'-diméthyl-1,1'-binaphtyle et le solvant dans le rapport massique 1:(3 à 40).

3. Procédé selon la revendication 2, caractérisé en ce que l'on utilise le 2,2'-diméthyl-1,1'-binaphtyle et le solvant dans le rapport massique 1:(4 à 20).

4. Procédé selon la revendication 3, caractérisé en ce que l'on utilise le 2,2'-diméthyl-1,1'-binaphtyle et le solvant dans le rapport massique 1:(5 à 15).

5. Procédé selon une ou plusieurs des revendications 1 à 4, caractérisé en ce que l'on utilise comme solvant un benzène chloré une ou plusieurs fois, un hydrocarbure aliphatique chloré une ou plusieurs fois, un ester d'un acide carboxylique aliphatique ayant 1 à 6 atomes de carbone et d'un alkylalcool ayant 1 à 4 atomes de carbone ou des mélanges de ceux-ci.

6. Procédé selon une ou plusieurs des revendications 1 à 5, caractérisé en ce que l'on utilise comme solvant le chlorobenzène ou le dichlorobenzène.

7. Procédé selon une ou plusieurs des revendications 1 à 6, caractérisé en ce que l'on utilise comme solvant un mélange de chlorobenzène et de dichlorobenzène.

8. Procédé selon une ou plusieurs des revendications 1 à 7, caractérisé en ce que l'on fait réagir le 2,2'-diméthyl-1,1'-binaphtyle et l'agent bromant sous l'action d'une lumière d'une longueur d'onde de 10⁻⁵ à 10⁻⁸ m à -10 à 75°C.

9. Procédé selon la revendication 8, caractérisé en ce que l'on fait réagir le 2,2'-diméthyl-1,1'-binaphtyle et l'agent bromant sous l'action d'une lumière d'une longueur d'onde de 10⁻⁵ à 10⁻⁸ m à -5 à +50°C.

10. Procédé selon la revendication 9, caractérisé en ce que l'on fait réagir le 2,2'-diméthyl-1,1'-binaphtyle et l'agent bromant sous l'action d'une lumière d'une longueur d'onde de 10⁻⁵ à 10⁻⁸ m à 0 à 40°C.

11. Procédé selon une ou plusieurs des revendications 1 à 10, caractérisé en ce que l'on fait réagir le 2,2'-diméthyl-1,1'-binaphtyle et l'agent bromant sous l'action d'un générateur de radicaux à 25 à 150°C.

12. Procédé selon la revendication 11, caractérisé en ce que l'on fait réagir le 2,2'-diméthyl-1,1'-binaphtyle et l'agent bromant sous l'action d'un générateur de radicaux à 40 à 135°C.

13. Procédé selon une ou plusieurs des revendications 1 à 12, caractérisé en ce que l'on fait réagir le 2,2'-diméthyl-1,1'-binaphtyle avec le N-bromosuccinimide comme agent bromant.

14. Procédé selon une ou plusieurs des revendications 1 à 13, caractérisé en ce que l'on utilise le 2,2'-diméthyl-1,1'-binaphtyle et le N-bromosuccinimide dans le rapport molaire 1:(1,5 à 2,5).

15. Procédé selon la revendication 14, caractérisé en ce que l'on utilise le 2,2'-diméthyl-1,1'-binaphtyle et le N-bromosuccinimide dans le rapport molaire 1:(1,8 à 2,3).

16. Procédé selon la revendication 15, caractérisé en ce que l'on utilise le 2,2'-diméthyl-1,1'-binaphtyle et le N-bromosuccinimide dans le rapport molaire 1:(1,9 à 2,2).

17. Procédé selon une ou plusieurs des revendications 8 à 16, caractérisé en ce que, après la bromation, on sépare le succinimide formé comme agent bromant qui a réagi.

18. Procédé selon une ou plusieurs des revendications 8 à 17, caractérisé en ce que l'on sépare par filtration le succinimide, éventuellement après refroidissement.

19. Procédé selon une ou plusieurs des revendications 8 à 18, caractérisé en ce que l'on sépare le succinimide par extraction à l'eau.

20. Procédé selon une ou plusieurs des revendications 8 à 18, caractérisé en ce que l'on sépare le succinimide dans une première étape par filtration et dans une deuxième étape par extraction à l'eau.

21. Procédé selon une ou plusieurs des revendications 1 à 20, caractérisé en ce que l'on fait réagir le mélange réactionnel avec le diphénylphosphinate d'alkyle de formule RO-P(Ph-(R')ₙ)₂ à 100 à 170°C.

22. Procédé selon la revendication 21, caractérisé en ce que l'on fait réagir le mélange réactionnel avec le diphénylphosphinate d'alkyle de formule RO-P(Ph-(R')ₙ)₂ à 120 à 160°C.

23. Procédé selon une ou plusieurs des revendications 1 à 22, caractérisé en ce que l'on utilise le diphénylphosphinate d'alkyle de formule RO-P(Ph-(R')ₙ)₂, par rapport au 2,2'-diméthyl-1,1'-binaphtyle utilisé, dans le rapport molaire (1,8 à 4):1.

24. Procédé selon la revendication 23, caractérisé en ce que l'on utilise le diphénylphosphinate d'alkyle de formule RO-P(Ph-(R')ₙ)₂, par rapport au 2,2'-diméthyl-1,1'-binaphtyle utilisé, dans le rapport molaire (1,9 à 3):1.

25. Procédé selon la revendication 24, caractérisé en ce que l'on utilise le diphénylphosphinate d'alkyle de formule RO-P(Ph-(R')ₙ)₂, par rapport au 2,2'-diméthyl-1,1'-binaphtyle utilisé, dans le rapport molaire (2 à 2,5):1.

26. Procédé selon une ou plusieurs des revendications 1 à 25, caractérisé en ce que l'on utilise comme diphénylphosphinate d'alkyle de formule RO-P(Ph-(R')ₙ)₂ un diphénylphosphinate d'alkyle en C₁-C₅ ou des mélanges de ces esters.

27. Procédé selon une ou plusieurs des revendications 1 à 26, caractérisé en ce que l'on utilise comme diphénylphosphinate d'alkyle de formule RO-P(Ph-(R')ₙ)₂ le diphénylphosphinate de méthyle, le diphénylphosphinate d'éthyle, le diphénylphosphinate d'isopropyle, le diphénylphosphinate de n-propyle, le bis-(3-fluorophényl)phosphinate d'éthyle, le bis-(4-fluorophényl)-phosphinate d'éthyle, le bis-(2-méthylphényl)phosphinate d'éthyle et/ou le bis-(3-trifluorométhylphényl)phosphinate d'éthyle.

28. Procédé selon une ou plusieurs des revendications 1 à 27, caractérisé en ce que, avant la réaction ou pendant la réaction ou après la réaction du mélange réactionnel avec le diphénylphosphinate d'alkyle, on ajoute un autre solvant qui bout à plus haute température que le solvant utilisé initialement, et on élimine par distillation le solvant utilisé initialement.

29. Procédé selon une ou plusieurs des revendications 1 à 28, caractérisé en ce que, pendant la réaction du mélange réactionnel avec le diphénylphosphinate d'alkyle, on ajoute comme autre solvant le o-xylène, le m-xylène, le p-xylène, des mélanges de ces xylènes et/ou le mésitylène et on élimine par distillation le chlorobenzène et/ou le dichlorobenzène comme solvant utilisé initialement.

30. Composés de formule 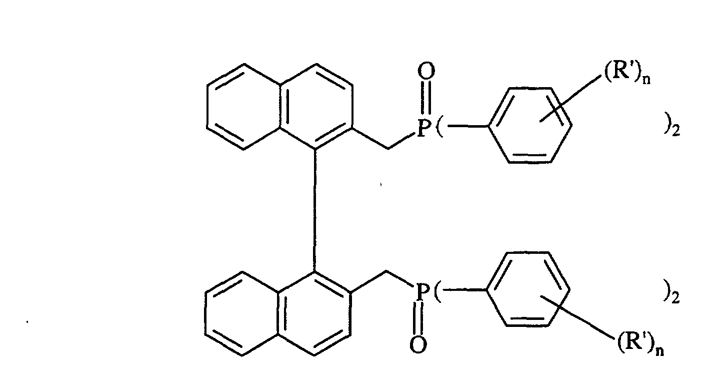 où R' représente un reste alkyle ayant 1 à 4 atomes de carbone, CF₃, le fluor, le chlore ou le brome et n représente 1 ou 2.

31. Composés de formule 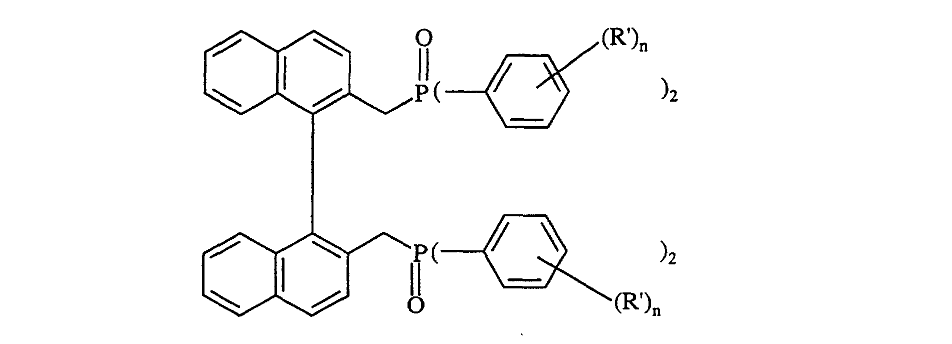 où R' représente méthyle, CF₃ ou le fluor et n représente 1 ou 2.

32. Les composés 2,2'-bis-[bis(3-fluorophényl)phosphinylméthyl]-1,1'-binaphtyle, 2,2'-bis-[bis(4-fluorophényl)phosphinylméthyl]-1,1'-binaphtyle, 2,2'-bis-[bis(2-méthylphényl)phosphinylméthyl]-1,1'-binaphtyle et 2,2'-bis-[bis(3-trifluorométhylphényl)phosphinylméthyl]-1,1'-binaphtyle.
